# EUROPEAN PATENT APPLICATION

(11) **EP 3 862 087 A1**
(43) Date of publication of application: **11.08.2021**
(21) Application number: 20156483.8
(22) Date of filing: 10.02.2020
(51) Int. Cl.: B01L 3/00, C12Q 1/6806, B01L 7/00

(54) **DEVICE AND METHOD FOR ANALYZING BIOLOGICAL SAMPLES**

(71) Applicant: diamond invention UG (haftungsbeschränkt), DE-14548 Schwielowsee (DE)
(72) Inventor: HEINSOHN, Natasha, 12524 Berlin (DE); ANIELSKI, Alexander, 14473 Potsdam (DE); NIEDL, Robert, 14089 Berlin (DE)
(74) Representative: Schulz Junghans Patentanwälte PartGmbB

(57) **Abstract**

The invention relates to a device (1) for analyzing biological samples (S) comprising a substrate (2) for receiving a biological sample (S), wherein the substrate (2) comprises or consists of a fibrous material (F) configured to form a stationary phase which retains molecules in a biological sample (S) dissolved in a liquid mobile phase depending on molecular weight and/or polarity of the molecules, a first electrode (41) and a second electrode (42), which are arranged along a first axis (A1) and configured to generate an electric field acting along the first axis (A1) when an electric potential difference is provided between the first electrode (41) and the second electrode (42), so that charged molecules contained in the biological sample (S) are movable through the substrate (2) along the first axis (A1) and/or separable by their molecular weight, their polarity and/or their charge, wherein the substrate (2) comprises a chemical lysing agent (L) capable of lysing the biological sample (S).

Furthermore, a method for analyzing biological samples (S) using the device (1) is provided.

## Description

The invention relates to a device and a method for analyzing biological samples, for example environmental samples (e.g. environmental water samples) or samples from human or animals (e.g. blood, saliva or sputum). In particular, the device and the method may be used to identify, and optionally taxonomically classify, pathogens (e.g. bacteria, viruses, fungi, protozoa, Helminthes or Arthropoda) which are present in the biological samples. These pathogens may be identified, in particular, by analyzing characteristic nucleic acids or proteins.

WO 2013/094316, WO 2014/025500 and WO 2017/040947 describe devices of the prior art capable of analyzing such biological samples to identify pathogens.

WO 2013/094316 discloses a micro/nanofluidic analyzer in which the vertical or horizontal movement of DNA within a channel can be controlled, the device comprising an electroconductive film provided on a substrate, a first electrode for applying a voltage to the electroconductive film, a groove part serving as a channel for a solution from the end face on one side of a first insulating film provided on the conductive film to the end face on the other side thereof, the upper face of the channel being open, at least one pair of electrode terminals provided opposing both side surfaces of the groove part, and second and third electrodes for applying a voltage to a wire connected to each of the electrode terminals. The device is configured such that the sample can be analyzed by measuring the electric signal between the second and third electrodes.

In WO 2014/025500, an automated microfluidic chip for biological analysis is described. A sample can be introduced via a sample acceptor to a first domain of the chip containing a pre-loaded liquid extraction solution comprising thermostable proteinases for DNA extraction. The chip further comprises a side channel to a second domain containing PCR reagents, and a fraction of the extracted DNA sample can be amplified in a PCR reaction chamber of the chip. After amplification, the DNA solution can be subjected to capillary electrophoresis and detected by a DNA analyzer.

WO 2017/040947 describes a device suitable to electrochemically analyze DNA comprising a substrate consisting of a porous cellulose matrix and a hydrophobic barrier defining a channel in the substrate and an electrically conducting material disposed within a volume of the channel.

These solutions according to the prior art have the disadvantage that samples must be externally and manually processed before being analyzed and/or that additional devices, such as pumps, are necessary to control liquid flow through the respective device. Furthermore, optical devices, such as microscopes or photometers, are required for certain analysis steps.

Accordingly, the objective underlying the present invention is to provide a device and a method for analyzing a biological sample which is improved in view of the above-discussed disadvantages of the prior art. In particular, the objective of the invention is to provide a device for analyzing a biological sample capable of taking up a liquid biological sample, automatically extracting biomolecules (e.g. DNA, RNA or proteins) from cells in the sample, optionally amplifying the biomolecules, automatically detecting the biomolecules, and feeding digital data from sample detection to a software for identifying the biomolecules.

This objective is attained by the subject matter of the independent claims 1 and 15. Embodiments of the invention are claimed as sub claims 2-14 and described hereafter.

A first aspect of the invention relates to a device for analyzing biological samples, the device comprising a substrate for receiving a biological sample, wherein the substrate comprises or consists of a fibrous material configured to form a stationary phase which retains molecules in a biological sample dissolved in a liquid mobile phase depending on molecular weight and/or polarity of the molecules.

The device further comprises at least two electrodes (particularly a first electrode and a second electrode), the electrodes being arranged along a first axis and configured to generate an electric field acting along the first axis when an electric potential difference (i.e. a voltage) is provided between the electrodes (particularly the first electrode and the second electrode), so that charged molecules (e.g. DNA, RNA and/or proteins) contained in the biological sample are movable (particularly electrophoretically movable) through the substrate along the first axis and/or separable (particularly electrophoretically) by their molecular weight, their polarity and/or their charge.

Further, according to the invention, the substrate comprises a chemical lysing agent capable of lysing the biological sample.

In the context of the present specification, the term *biological sample* designates a sample containing organisms (i.e., prokaryotes and/or eukaryotes) or a sample obtained from an organism, such as a human or an animal. In particular, the biological sample contains biomolecules.

The term *biomolecule,* as used herein, describes a molecule produced by a living organism, for example deoxyribonucleic acids (DNA), ribonucleic acids (RNA), proteins, carbohydrates, lipids, metabolites or other small molecules.

A *fibrous material* in the context of the present specification is a material comprising or consisting of fibers (i.e. elongated chains of material), in particular having pores between the fibers, more particularly having an average pore size of 100 nm to 1000 nm, more particularly 500 nm to 900 nm, most particularly about 700 nm.

The substrate is able to retain molecules of the biological sample dissolved in a liquid mobile phase depending on its molecular weight and/or polarity (their molecular weight, their polarity or both) according to the chromatographic effect, that is in the presence or absence of an additional electric field generated by the electrodes of the device. In particular, the ability to retain dissolved molecules according to molecular weight depends on the pore size of the fibrous material, and the ability to retain dissolved molecules according to polarity depends on the hydrophobicity and/or the charge of the fibrous material. In particular, the polarity of the molecules in the biological sample depends on their hydrophobicity and/or charge (i.e., in some cases, molecules may be hydrophobic, but comprise a charge at the same time, e.g. trimethyl ammonium).

In the context of the present specification, the term *polarity* describes a charge separation in groups of atoms resulting in the group of atoms no longer being electrically neutral. In particular, the polarity may be quantified by the electric dipole moment.

The electrodes of the device may comprise any shape and may be formed from any suitable, i.e. electrically conductive material.

The electrophoretic mobility of molecules of the biological sample in the electric field generated by the electrodes depends on their net charge in the used solvent (i.e. buffer) and their molecular weight, whereas larger molecules migrate slower than smaller molecules and highly charged molecules migrate faster than less charged molecules. In addition, depending on the hydrophobicity of the fibrous material, the mobility of the molecules in the substrate may also depend on their polarity.

The terms *lyse* or *lysis* as used herein designate the perforation of the plasma membrane of biological cells, which particularly leads to their destruction and the release of cellular biomolecules. A *chemical lysing agent* in the context of the present specification is a chemical which is capable of lysing biological cells.

In certain embodiments, the chemical lysing agent comprises an alkaline compound, particularly provided in an amount sufficient to result in a pH of 11 or more in the biological sample when the biological sample is brought in contact with the chemical lysing agent on the substrate. For example, the alkaline compound is NaOH or KOH.

In certain embodiments, the chemical lysing agent comprises an amphiphilic compound, e.g. a detergent or a surfactant.

In particular, the amphiphilic compound is selected from sodium dodecyl sulfate (SDS, CAS number 151-21-3), 3 - [(3-Cholamidopropyl) dimethylammonio]-1-propanesulfonate (CHAPS, CAS number 75621-03-3), 3-([3-Cholamidopropyl]dimethylammonio)-2-hydroxy-1-propanesulfonate (CHAPSO, CAS number 82473-24-3), 2-[4-(2,4,4-trimethylpentan-2-yl)phenoxy]ethanol (Triton-X, CAS number 9002-93-1), a polysorbate, more particularly polysorbate 20 (polyoxyethylen(20)-sorbitanmonolaurat, CAS number 9005-64-5) or polysorbate 80 (polyoxyethylen(20)-sorbitanmonooleat, CAS number 9005-65-6).

In certain embodiments, the chemical lysing agent comprises a chelating agent, particularly ethylenediamin-tetra acetic acid (EDTA, CAS number 60-00-4). A chelating agent as used herein is a ligand capable of forming coordinate bonds with a central atom, particularly a metal.

In certain embodiments, the chemical lysing agent comprises lysozyme. Lysozyme is an enzyme capable of hydrolyzing b-1,4 glycoside bonds between N-acetylmuramic acid and N-acetyl-D-glucosamine residues in peptidoglycan.

In certain embodiments, the chemical lysing agent comprises an amphiphilic compound, particularly CHAPS, and lysozyme.

In certain embodiments, the chemical lysing agent comprises an amphiphilic compound, particularly SDS, and an alkaline compound, particularly NaOH.

In certain embodiments, the chemical lysing agent comprises an amphiphilic compound and a chelating agent.

An advantage of the chemical lysing agent which is comprised in the substrate of the device is that no separate lysing step using additional chemicals and/or external devices (i.e. French press, sonicator) are necessary to provide the biomolecules of interest which are to be analyzed. Thereby, the device according to the invention reduces preparation time and saves costs for additional chemicals and external devices. In addition, the device is usable without external electrodes. Combined with the integrated lysing agent, this results in improved usability of the device especially for on-site analysis outside of a laboratory.

In addition to the effect of the chemical lysing agent, biological cells may also be lysed physically in the device according to the invention, e.g. by the properties of the fibrous material onto which they are applied. For example, the drying of the biological sample on the substrate may induce cell lysis by osmotic shock. This is especially advantageous in combination with chemical lysing agents comprising amphiphilic compounds and chelating agents, which supports induced membrane perforation by osmotic shock.

The device functions without external pumps and the chromatographic effect of the fiber material is applied for the sized-dependent separation of samples. Cell debris and particularly undesired protein contamination in case of DNA, can be removed by a coarse filtering effect of the fiber material. An electrical field is then applied for fine separation of the molecules to be analyzed. In this step, the biomolecules are actively drawn through the liquid-permeated fiber network. Notably, the biomolecules may be separated without (active) liquid flow and may be isolated at specific locations in the sample channel formed in the fibrous substrate.

In certain embodiments, the device is a portable device. In other words, the device is configured to be carried by hand and can be easily transported to sites outside of the laboratory for on-site analysis.

In certain embodiments, the first axis extends vertically in an operational configuration of the device.

In certain embodiments, the first axis extends diagonally (in respect of a vertical direction) in an operational configuration of the device. This has the advantage that the biological sample is separated such that bands of molecules in the biological sample are aligned next to each other in a projection along a horizontal plane when the bands are detected optically.

In certain embodiments, the fibrous material is selected from glass fiber, polyamide, cellulose, nitrocellulose, viscose, PEG, acrylamide or chitosan.

In certain embodiments, the lysing agent is disposed within the substrate in dry form, wherein the lysing agent is dissolvable upon applying a liquid biological sample to the substrate, such that the biological sample is lysed.

In particular, this increases the time that the device can be stored before use.

In certain embodiments, the lysing agent is disposed in a lysis reservoir in the substrate, that is in a hollow space in the fibrous material.

In certain embodiments, the substrate comprises a barrier structure which is impermeable to the liquid mobile phase, wherein the barrier structure forms at least one channel which is permeable to the liquid mobile phase in the substrate limited by the barrier structure. In particular, in case of an aqueous mobile phase, the barrier structure is water-impermeable. In particular, in case of a hydrophobic mobile phase (e.g. based on an organic solvent such as an oil or benzene), the barrier structure is hydrophilic.

In the context of the present specification, the term 'permeable' refers to the ability of a part or material to be wetted and/or permeated by a liquid substance.

In certain embodiments, the barrier structure comprises a wax, a plastic (particularly plastic foil), a varnish, a glue, or an impregnating agent, particularly a hydrocarbon, more particularly an oil.

In certain embodiments, the substrate comprises a partial area of fibrous material which is permeable to the liquid mobile phase (particularly water-permeable) surrounded by the barrier structure, wherein particularly the partial area is perpendicular to the first axis.

In certain embodiments, the device comprises a third electrode, wherein the second electrode and the third electrode are arranged along a second axis which is non-parallel to the first axis, and wherein the second electrode and the third electrode are configured to generate an electric field acting along the second axis when an electric potential difference (i.e., a voltage) is applied between the second electrode and the third electrode.

In certain embodiments, the first electrode, the second electrode and/or the third electrode is embedded into the fibrous material of the substrate or connected to the fibrous material of the substrate.

This advantageously obviates the need to assemble the substrate with separate electrodes during manufacture, and thereby simplifies manufacture of the device.

In certain embodiments, the first electrode, the second electrode and/or the third electrode is printed onto the substrate.

By printing methods with an electrically conductive ink, the device may be easily produced at low costs. In addition, by printing, the electrodes may be placed between different layers of the fibrous material, which is of advantage if the electric signal (particularly current or voltage over time) is used to analyze the separated components (see below)
In certain embodiments, the second electrode comprises a cross-sectional area perpendicular to the first axis, which is smaller than a cross-sectional area perpendicular to the first axis of the first electrode, so that charged molecules in the biological sample are focused in the substrate while moving through the electric field between the first electrode and the second electrode. Therein, the cross-sectional area of the electrode influences the electric field vectors between the first and the second electrode. Due to the smaller cross-sectional area of the second electrode, the electric field converges towards the second electrode, such that charged molecules are focused.

In certain embodiments, the first electrode forms a grid structure, particularly extending at least partially over the partial area of the substrate which is permeable to the liquid mobile phase (particularly water-permeable), surrounded by the barrier structure.

In certain embodiments, the grid structure is perpendicular to the first axis.

In certain embodiments, the grid structure is formed by a plurality of rods arranged in parallel.

In certain embodiments, the first electrode, the second electrode and/or the third electrode are connected to an outside of the device by a respective conductive contact tab, wherein an end of the contact tab is configured to be electrically connected to a pole of a voltage source.

In certain embodiments, the first electrode is connected to an outside of the device by a conductive contact tab, wherein an end of the contact tab is configured to be electrically connected to a pole of a voltage source.

In certain embodiments, the second electrode is connected to an outside of the device by a conductive contact tab, wherein an end of the contact tab is configured to be electrically connected to a pole of a voltage source.

In certain embodiments, the third electrode is connected to an outside of the device by a conductive contact tab, wherein an end of the contact tab is configured to be electrically connected to a pole of a voltage source.

In certain embodiments, a DNA polymerase and PCR primers (in other words, PCR reagents) are disposed in the substrate, so that a DNA sequence of the biological sample can be amplified by PCR by means of the DNA polymerase if the PCR primers contain complementary DNA sequences to the DNA sequence.

In the context of the present specification, the term *PCR* designates the method polymerase chain reaction, wherein DNA is amplified from a DNA or RNA template using PCR primers (single-stranded oligonucleotides typically having a length of 15-35 bases) and a DNA polymerase (e.g. Taq or Pfu polymerase).

The DNA polymerase may be a DNA dependent or RNA dependent DNA polymerase, in other words may be capable of using DNA or RNA as a template for DNA polymerization.

In certain embodiments, deoxy ribonucleotides, particularly a mix of deoxy adenosine triphosphate (dATP), deoxy cytidine triphosphate (dCTP), deoxy guanosine triphosphate (dGTP) and deoxy thymidine triphosphate (dTTP) are disposed in the substrate. These deoxy ribonucleotides serve as a substrate for the DNA polymerase to amplify DNA in the porous substrate of the device.

In certain embodiments, the device comprises a polymer gel, particularly a hydrogel, in contact with the substrate, so that liquid is exchangeable between the polymer gel and the substrate.

In certain embodiments, the polymer gel comprises acrylamide, acrylic acid, N-isopropyl acrylamide (NIPAM, CAS number 2210-25-5), or a carbohydrate, particularly agarose.

In certain embodiments, the polymer gel is switchable by setting a physical parameter, so that liquid is exchanged between the polymer gel and the substrate.

The liquid exchanged between the substrate and the polymer gel may be applied in particular to draw the biological sample through the substrate or to prevent drying of the substrate, particularly during heating of the substrate when a PCR is performed in the substrate.

In certain embodiments, setting the physical parameter is changing a temperature, changing a pH, changing an ionic strength, providing electromagnetic radiation, or initiating a chemical reaction.

In certain embodiments, the device comprises a heating element for heating, particularly periodic heating, of the substrate. In particular, the heating element is configured to periodically heat the PCR reagents disposed in the substrate (e.g. up to about 60 times). In particular, the heating element is configured to heat the polymer gel, such that liquid is exchanged between the polymer gel and the substrate.

An integrated heating element has the advantage that functions such as performing a PCR and controlling / switching a polymer gel can be performed without external devices, improving the usability of the device in settings outside of the laboratory.

In certain embodiments, the heating element is formed as a heating electrode.

In certain embodiments, the heating electrode is connected to an outside of the device via electrically conductive first and second contact tabs, wherein ends of the first and second contact tabs are configured to be electrically connected to the poles of a voltage source, so that an electric current flows through the heating electrode thereby heating the substrate by its electric resistance.

In certain embodiments, the heating element is embedded in the fibrous material or placed adjacent to the fibrous material.

This advantageously simplifies the manufacture of the device.

In particular, by periodic heating of the substrate using the heating element, a DNA sequence of the biological sample can be amplified by the DNA polymerase and PCR primers if the PCR primers contain complementary DNA sequences to the DNA sequence. Alternatively or additionally, a switchable polymer gel contained in the substrate may be switched by heating using the heating element.

In certain embodiments, the device comprises a cooling element for periodic cooling of the substrate. The cooling element may be separate from the heating element.

In certain embodiments, the device comprises a heating-and-cooling element, in other words the element is configured to heat and cool, particularly actively cool, the substrate. In particular, the device is configured to periodically heat and cool the substrate.

Cooling the substrate is especially advantageous when a PCR is performed in the substrate. To this end, the heating element and the cooling element or the combined heating-and-cooling element are/is configured to periodically cycle between a melting temperature (e.g. 94 °C to 98 °C) at which DNA secondary structures are melted, an annealing temperature (e.g. 45 °C to 65 °C) at which the PCR primers anneal to complementary DNA or RNA sequences of the template DNA and an elongation temperature (e.g. 68 °C to 72 °C) at which the DNA polymerase polymerizes DNA. A cooling device improves the speed of cycling between these temperatures.

In certain embodiments, the heating-and-cooling element is a Peltier element configured to periodically heat and cool the substrate.

In certain embodiments, the substrate comprises a marker for labeling molecules contained in the biological sample, particularly wherein the marker comprises a dye or wherein the marker is detectable by its redox properties (referred to hereafter as a redox marker).

In certain embodiments, the redox marker is a quinone, ferrocen (Bis (η⁵-cyclopentadienyl) iron, CAS number 102-54-5), tetrathiafulvalen (CAS number 31366-25-3), a ruthenium-terpyridine complex, cobaltocen (Bis(cyclopentadienyl) cobalt, CAS number 1277-43-6), potassium hexa cyano ferrate (e.g. CAS number 13943-58-3), or pyrroloquinolin quinon (CAS number 72909-34-3) .

In certain embodiments, these compounds can be applied in combination with enzymes such as a peroxidase, particularly horse radish peroxidase, an oxidase, particularly glucose oxidase, or a phosphatase, particularly alkaline phosphatase, wherein the enzyme degrades the redox marker, and wherein the redox potential resulting from the redox marker is measured by the first electrode and the second electrode.

In certain embodiments, the redox marker is ferrocen and the enzyme is a peroxidase, particularly horseradish peroxidase.

In certain embodiments, the redox marker is pyrroloquinolin quinon and the enzyme is glucose oxidase-FADH₂ (glucose oxidase bound to FADH₂).

In certain embodiments, the marker is disposed in a marker reservoir or a sensor reservoir in the substrate.

In certain embodiments, the marker is antibody, an aptamer, a peptide, or a hybridization probe.

The advantage of a marker integrated into the device is that no separate labeling step and no additional components are necessary.

In the context of the present specification, the term *hybridization probe* describes an oligonucleotide capable of hybridizing to single stranded DNA or RNA.

In certain embodiments, the hybridization probe comprises a loop structure of single stranded DNA or RNA and a stem structure of double stranded DNA or RNA, wherein the stem is formed by annealing of complementary nucleotide sequences at the 5' and 3' ends of the hybridization probe. In particular, one end of the hybridization probe may contain a fluorophore and the other end of the hybridization probe may contain a quencher capable of quenching the fluorescence of the fluorophore when the ends of the hybridization probe form the stem structure. In particular, when the loop structure hybridizes to a complementary DNA or RNA sequence, the stem structure is dissolved resulting in separation of the fluorophore and the quencher and therefore de-quenching of the fluorescence.

In the context of the present specification, the term aptamer describes a DNA molecule, RNA molecule or a peptide having a three-dimensional structure, wherein the DNA molecule, RNA molecule or peptide is capable of specifically binding a target molecule due to its three-dimensional structure.

In certain embodiments, the device is at least partially transparent, so that the molecules contained in the biological sample can be analyzed by an optical detector.

In certain embodiments, the optical detector is a camera, particularly a camera contained in a handheld device such as a smart phone or a tablet computer.

In certain embodiments, the device comprises a plurality of layers comprising the fibrous material, wherein the layers are arranged on top of each other (e.g. stacked along a vertical axis), so that the fibrous material of the layers forms the substrate wherein the layers extend in respective planes which are non-parallel to the first axis, so that charged molecules contained in the biological sample are movable through the layers by means of the electric field acting along the first axis between the first electrode and the second electrode, particularly wherein the planes in which the layers extend, are parallel to the second axis, so that the charged molecules contained in the biological sample are movable in the respective layer and separable by their molecular weight, polarity and/or charge.

In other words, the first electrode and the second electrode are arranged such that components of the biological sample are moved through several layers, whereas the second and the third electrode (if applicable) are arranged such that the biological sample is moved in a layer.

Such a layer structure has the advantage that the device can be easily manufactured by producing and stacking the layers, and that a modular design can be achieved, meaning that certain layers may be selected, combined or changed according to the need of the user.

The fibrous material of the layers jointly forms the substrate. In particular, this is achieved by the fibrous material of a respective layer being in contact with the fibrous material of the adjacent layer.

In certain embodiments, the device comprises a first layer (also termed lysis layer), wherein the fibrous material of the first layer comprises the lying agent, a second layer (also termed filter layer) in contact with the first layer, wherein the second layer is configured to filtrate the biological sample when the biological sample passes through the second layer, and a third layer (also termed analysis layer) in contact with the second layer.

The fibrous material, particularly of the filter layer, may comprise an (average) pore size of 100 nm to 1000 nm, more particularly 500 nm to 900 nm, most particularly about 700 nm.

In certain embodiments, the first layer comprises the first electrode, wherein the first electrode is particularly embedded, more particularly printed on the first layer.

In certain embodiments, the third layer comprises the second electrode, wherein the second electrode is particularly embedded, more particularly printed on the third layer.

In certain embodiments, the third layer further comprises the third electrode, such that molecules in the biological sample can be separated by molecular weight, polarity and/or charge in the third layer using the electric field acting between the second electrode and the third electrode. The third electrode may be embedded in, particularly printed on the third layer.

In certain embodiments, the first layer comprises a barrier structure, which is impermeable to the liquid mobile phase, particularly water-impermeable, wherein the barrier structure forms at least one channel which is permeable to the liquid mobile phase (particularly water-permeable) in the substrate, the at least one channel being limited by the barrier structure, particularly wherein the first layer comprises a first (partial) area of fibrous material which is permeable to the liquid mobile phase (particularly water-permeable) and surrounded by the barrier structure.

In certain embodiments, the second layer comprises a barrier structure, which is impermeable to the liquid mobile phase, particularly water-impermeable, wherein the barrier structure forms at least one channel which is permeable to the liquid mobile phase (particularly water-permeable) in the substrate, the at least one channel being limited by the barrier structure, particularly wherein the second layer comprises a second (partial) area of fibrous material which is permeable to the liquid mobile phase (particularly water-permeable) and surrounded by the barrier structure.

In certain embodiments, the third layer comprises a barrier structure, which is impermeable to the liquid mobile phase, particularly water-impermeable, wherein the barrier structure forms at least one channel which is permeable to the liquid mobile phase (particularly water-permeable) in the substrate, the at least one channel being limited by the barrier structure, particularly wherein the third layer comprises a third (partial) area of fibrous material which is permeable to the liquid mobile phase (particularly water-permeable) and surrounded by the barrier structure.

In certain embodiments, the fibrous material, particularly the third area, of the third layer comprises the marker and/or the DNA polymerase and the PCR primers (and particularly also the deoxy ribonucleotides).

In certain embodiments, the fibrous material, particularly the third area, of the third layer is in contact with the heating element.

In certain embodiments, the polymer gel is arranged in the first layer and/or the second layer.

In certain embodiments, the first layer comprises an opening for inserting a polymer gel. In certain embodiments, the second layer comprises an opening for inserting a polymer gel. In certain embodiments, the first layer and the second layer comprise respective openings, wherein the openings overlap when the first layer and the second layer are stacked, such that at least one polymer gel can be placed in the openings when the first layer and the second layer are stacked.

Therein, the first layer, the second layer and the third layer are comprised in the aforementioned plurality of layers.

In certain embodiments, the device further comprises at least one cover layer which is impermeable to the liquid mobile phase, wherein the cover layer is configured to cover the substrate, particularly the first layer or the third layer. In particular, the cover layer is formed from a foil material.

In certain embodiments, the cover layer comprises a first cover layer configured to cover the first layer and a second cover layer configured to cover the third layer.

In certain embodiments, the cover layer, particularly the first cover layer, comprises an opening overlapping, particularly congruent with, the first area of the first layer, when the cover layer and the first layer are stacked, such that a sample can be applied onto the substrate, particularly the first area, via the opening.

In certain embodiments, the cover layer, particularly the second cover layer comprises a heating element, particularly wherein the heating element is embedded in, more particularly printed on the cover layer. Therein, in particular, the heating element is a resistance heater, such as a heating electrode.

In certain embodiments, the device comprises a cover or a lid configured to cover the substrate, particularly the first layer or the third layer, or the cover layer.

In certain embodiments, the cover or lid is configured to cover the opening of the cover layer and/ or the first area of the first layer and/ or the third area of the third layer.

In certain embodiments, the cover or the lid comprises the first electrode. In particular, the first electrode forms a grid structure (see description above). In particular, the first electrode is embedded in, particularly printed on the cover or the lid.

A second aspect of the invention relates to a method for analyzing biological samples by means of the device according to the first aspect, wherein a biological sample is applied to the substrate, such that the biological sample is in contact with the lysing agent, resulting in lysis of the biological sample.

In certain embodiments, an electric potential difference (i.e. voltage) is provided between the first electrode and the second electrode, such that the biological sample moves through the substrate and is separated by its molecular weight, polarity and/or charge.

In certain embodiments, the substrate is periodically heated, such that a DNA sequence of the biological sample is amplified by PCR in the substrate.

In certain embodiments, an electric potential difference is provided between the second electrode and the third electrode, such that the biological sample is separated by its molecular weight, polarity and/or charge.

In certain embodiments, the current between the second electrode and the third electrode is measured over time, wherein properties of the biological sample are derived from the measured current. This method may be referred to as amperometry or amperometric analysis.

In certain embodiments, at least one additional electrode is provided between the second electrode and the third electrode, wherein a potential difference is provided between the second electrode and each additional electrode, wherein the current between the second electrode and the third electrode and the current between the second electrode and the at least one additional electrode is measured over time, and wherein properties of the biological sample are derived from the measured currents. This has the advantage that the time resolution is increased.

In certain embodiments, a fourth electrode, a fifth electrode and a sixth electrode is provided between the second electrode and the third electrode, wherein a potential difference is provided between the second electrode and the fourth electrode, the second electrode and the fifth electrode and the second electrode and the sixth electrode, and wherein the current between the second electrode and the third electrode, the second electrode and the fourth electrode, the second electrode and the fifth electrode and the second electrode and the sixth electrode is measured over time, and wherein properties of the biological sample are derived from the measured currents. This method may be referred to as five-electrode cyclic amperometry.

In certain embodiments, the voltage between the second electrode and the third electrode is measured over time, wherein properties of the biological sample are derived from the measured voltage. This method may also be referred to as cyclic voltametry.

In certain embodiments, the charge consumed or produced at the second electrode or the third electrode is measured, wherein properties of the biological sample are derived from the measured charge. This method may also be referred to as coulometry.

In certain embodiments, the impedance or the resistance between the second electrode and the third electrode is measured over time, wherein properties of the biological sample are derived from the measured impedance or resistance.

In other words, during the electrophoretic movement of the biomolecules, particularly DNA molecules, the electrodes and the liquid disposed in the fibrous material serve as a resistance in an electric circuit. Using suitable software, this can be applied to monitor the separation process, particularly as a characteristic current signal. From these characteristic molecule data, the amount and type of electrophoretically migrating molecules may be determined.

In certain embodiments, the biological sample is analyzed by detecting the marker in the substrate.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.
- Fig. 1: shows a perspective exploded view of a device according to a first embodiment of the invention;
- Fig. 2: shows a part of the device depicted in Fig. 1 illustrating details of the first layer, the second layer and the third layer;
- Fig. 3 to 6: illustrate different modes of operation of the device according to the first embodiment of the invention;
- Fig. 7: illustrates optical detection of molecules in the biological sample on the substrate of the device according to the invention;
- Fig. 8: shows a further embodiment of the device according to the invention comprising a lid including the first electrode;
- Fig. 9: shows a further embodiment of the device according to the invention comprising resistance heating elements disposed on a cover layer;
- Fig. 10: shows a further embodiment of the device according to the invention comprising resistance heating elements disposed on fourth layer,
- Fig. 11: shows a further embodiment of the device according to the invention realizing a diagonal sample path through the substrate layers between the first electrode and the second electrode;

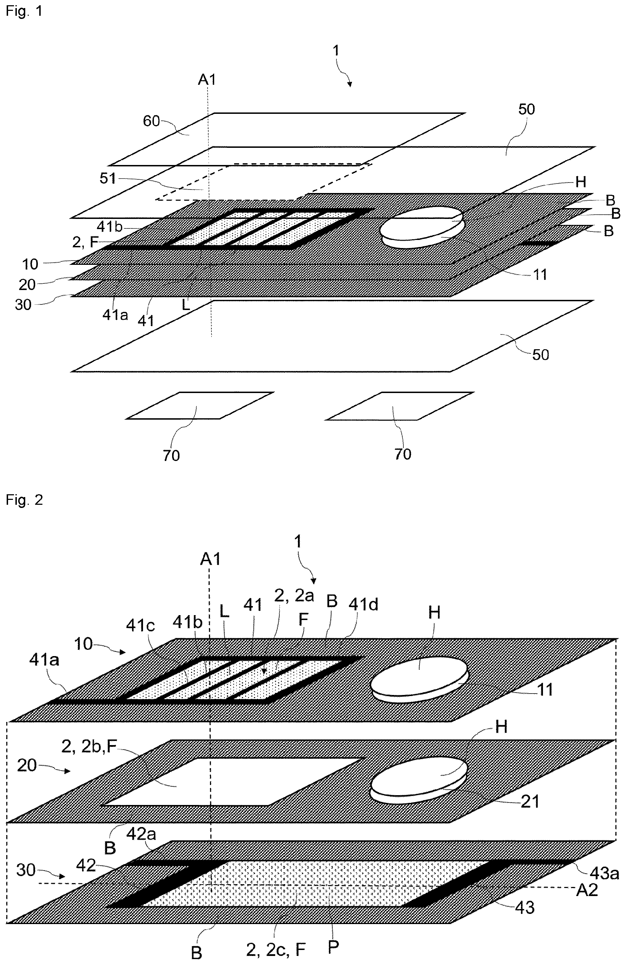

Fig. 1 shows a device 1 according to the present invention comprising a plurality of layers stacked along a first axis A1. The device 1 comprises from top to bottom according to the view of Fig. 1: a cover 60, a cover layer 50, a first layer 10, a second layer 20 and a third layer 30, each formed from the fibrous material F with an additional barrier structure B, and a further cover layer 50.

In the example described here, the liquid mobile phase to be used with the device 1 is particularly aqueous. Consequently, the fibrous material F is water-permeable, wherein the fibrous material F is particularly paper. In addition, the cover 60, the cover layers 50, and the barrier structure B is particularly water-impermeable. Of course, if a hydrophobic liquid mobile phase (e.g. based on an organic solvent) is to be used with the device 1, the fibrous material F is particularly hydrophobic (and therefore permeable to the hydrophobic liquid mobile phase), and the cover 60, the cover layers 50 and the barrier structure B are particularly hydrophilic or water-permeable (and therefore impermeable to the hydrophobic liquid mobile phase).

The cover 60 and the two cover layers 50 protect the first layer 10, the second layer 10, and the third layer 30 from ingress of water and contaminations, particularly during transport and storage of the device 1. The upper cover layer 50 comprises an opening 51 (particularly having a rectangular shape) through which the biological sample S may be applied to the first layer 10 after the cover 60 has been removed.

In the embodiment depicted in Fig. 1, the first layer 10 comprises a first area of fibrous material F (particularly having a rectangular shape) surrounded by the barrier structure B (the barrier structure B is displayed crosshatched). This fibrous material F forms a part of the substrate 2 as specified below. When the upper cover layer 50 is placed on top of the first layer 10, the opening 51 of the upper cover layer 50 is congruent with the first area of accessible (i.e., not being covered by the barrier structure B) fibrous material F of the first layer 10.

Fig. 1 further displays heating elements 70 positioned below the lower cover layer 50. During operation of the device 1, the lower cover layer 50 is particularly in direct contact with the lower cover layer 50, and the lower cover layer 50 is in direct contact with the third layer 30, such that the third layer 30 may be heated by the heating elements 70.

Fig. 2 shows details of the first layer 10, the second layer 20 and the third layer 30 of the device 1, omitting the cover layers 50, cover 60 and heating elements 70 for clarity.

As shown in Fig. 2, the first area 2a of fibrous material F of the first layer 10 comprises a chemical lysing agent L which is particularly embedded into and essentially equally distributed throughout the fibrous material F of the first layer 10. The lysing agent L is capable of lysing cells (bacteria, archaea or eukaryotes) contained in the biological sample S, e.g. by denaturation of lipid membranes and/or degradation of proteins which are part of the cell structure. In particular, the lysing agent L is disposed in the fibrous material F in dry or lyophilized form, such that the lysing agent L is dissolved when liquid is added, e.g. by application of the liquid biological sample S or by release of the liquid from a switchable polymer gel H (see below).

The first layer 10 further comprises a first electrode 41 shaped as grid structure 41b which comprises parallel rods or bars 41c from an electrically conductive material extending in the plane of the first layer 10. The bars 41c are surrounded by a frame 41d (particularly having a rectangular shape) arranged around the first area 2a of accessible fibrous material F. A contact tab 41a, particularly from the same electrically conductive material, connects the electrode 41, particularly the frame 41d, to an edge of the first layer 10, such that the contact tab 41 can be electrically connected to a positive or negative pole of an external voltage source. The electrode 41 is particularly printed onto the fibrous material F of the first layer 10, i.e. by an electrically conductive ink.

Furthermore, the first layer 10 comprises a circular opening 11 harboring a switchable polymer gel H. The opening 11 is separated from the first area 2a of accessible fibrous material F forming part of the substrate 2 by the barrier structure B, such that liquid released from the polymer gel H cannot access the substrate 2 via the first layer 10.

Similar to the first layer 10, the second layer 20 comprises a second area 2b of uncoated, accessible fibrous material F (particularly having a rectangular shape) surrounded by the barrier structure B, the uncoated fibrous material F of the second area 2b forming a part of the substrate 2. Said second area 2b of the second layer 20 is congruent with the first area 2a of the first layer 10 when the first layer 10 and the second layer 20 are placed on top of each other. The second layer 20 also comprises a circular opening 21 which is congruent with the opening 11 of the first layer 10 when the first layer 10 and the second layer 20 are placed on top of each other. The opening 21 may harbor a further polymer gel H, or the openings 11, 21 of the first layer 10 and the second layer 20 may jointly harbor a polymer gel H.

The third layer 30 comprises a third area 2c of freely accessible (i.e. uncovered by the barrier structure B) fibrous material F (particularly having a rectangular shape) surrounded by the barrier structure B. The third area 2c is larger (extends further to the right in Fig. 2) than the first area 2a of the first layer 10 and the second area 2b of the second layer 20. When the first layer 10, the second layer 20 and the third layer 30 are stacked on top of each other, the third area 2c overlaps with the first area 2a of the first layer 10 and the second area 2b of the second layer 20, such that the three areas 2a, 2b, 2c of fibrous material F jointly form the substrate 2 providing a channel through which the biological sample S may migrate as specified below.

The fibrous material F of the third area contains PCR reagents P (i.e. a DNA polymerase, PCR primers and deoxy ribonucleotides), which are particularly provided in dry or lyophilized form and are particularly equally distributed in the fibrous material F of the third layer 30. Thereby, if a liquid biological sample S containing DNA or RNA with sequences complementary to the PCR primers provided in the fibrous material enters the fibrous material F of the third layer 30, and if the third area 2c of fibrous material F is periodically heated and cooled to appropriate melting, annealing and elongation temperatures, the complementary DNA or RNA sequence may be amplified by PCR within the fibrous substrate F.

Furthermore, the third layer 30 comprises a second electrode 42 and a third electrode 43 which are arranged on opposite edges of the third area 2c and connected to respective outer edges of the third layer 30 by contact tabs 42a, 43a, such that the second electrode 42 and the third electrode 43 may be electrically connected to poles (particularly opposite poles of an external voltage source via the contact tabs 42a, 43a.

Moreover, when the first layer 10, the second layer 20 and the third layer 30 are placed on top of each other, the polymer gel H or polymer gels H disposed in the openings 11, 21 of the first layer 10 and the second layer 20 are in contact with the third area 2c of the third layer 30, such that liquid may be released from the polymer gel(s) H into the fibrous material F of the third layer 30, and/or such that liquid may be withdrawn from the fibrous material F of the third layer 30 into the polymer gel(s) H, as further specified below.

In particular, to manufacture the layers 10, 20, 30, thin sheets of the fibrous material F are provided and optionally cut into an appropriate size. Subsequently, the layers are particularly coated with a hydrophobic material, such as wax, to form the barrier structure B, and the openings 11, 21 of the first and second layer 10, 20 are particularly cut out. The first electrode 41, the second electrode 42 and the third electrode 43 may then be printed onto the first layer 10 and the third layer 30 using an electrically conductive ink. Finally, the lysing agent L may be applied to the first layer 10, particularly by applying a liquid solution containing the lysing agent L onto the first rectangular area 2a, and subsequently drying the first layer 10. Likewise, the PCR reagents P may be applied onto the third area 2c of the third layer as a liquid solution and subsequently dried. The layers 10, 20, 30 may then be stacked above each other, and the polymer gel H may be applied in the openings 11, 21 of the first layer 10 and/or second layer 20. Finally, the cover layers 50 and cover 60 may be placed on the stack as depicted in Fig. 1.

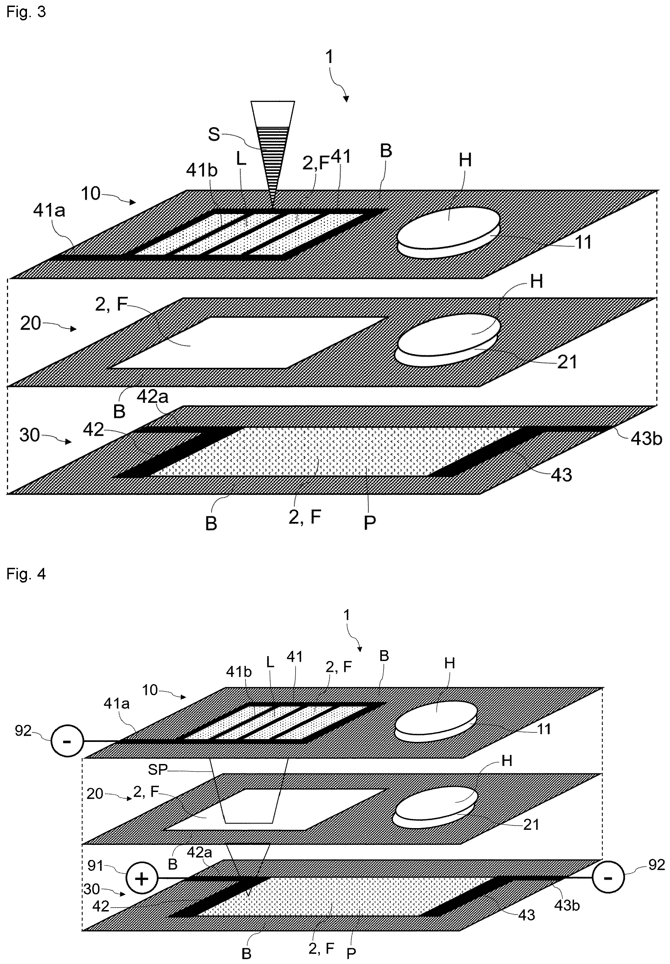

The use of the device 1 is described hereafter with reference to Figures 3-6.

As shown in Fig. 3, a liquid biological sample S is first applied to the first area 2a of the first layer 10, e.g. by manually or automatically pipetting the sample S onto the fibrous material F of the first area 2a. Thereby, in particular, the dry lysing agent L disposed in the fibrous material F is dissolved in the liquid biological sample S and cells in the biological sample S are lysed by the lysing agent L, thus releasing biomolecules such as DNA, RNA and proteins from these cells.

Since the first area 2a of fibrous material F in the first layer 10 is in contact with the second area 2b of the second layer 20 and the third area 2c of the third layer 30, the lysed liquid biological sample S is subsequently drawn into the fibrous material F of the second area 2b and the third area 2c by capillary forces. Therein, the liquid, in which the biological sample S is dissolved, serves as a mobile phase and the fibrous material F of the layers 10, 20, 30 serves as a stationary phase, whereby the fibrous material F, particularly the second area 2b of the second layer 20, filtrates the biological sample S by separating the components of the biological sample S by molecular weight due to the chromatographic effect. In this manner, in particular, larger particles, such as cell debris, may be separated from the biomolecules (i.e., DNA, RNA and proteins) of the biological sample, and particularly remain in the fibrous material F of the second layer 20. Optionally, the fibrous material F may also be adapted to separate the biological sample by polarity, depending on the hydrophobicity of the fibrous material F and the solvent of the biological sample S.

To facilitate the movement of the biological sample from the first layer 10 to the second layer 20 and the third layer 30, polymer gels H capable of swelling (taking up liquid) may be placed in the openings 11, 21 of the first and second layer 10, 20 to draw the solvent of the biological sample through the first layer 10, the second layer 20 and the third layer 30.

Alternatively or additionally, the device 1 may be placed in a liquid reservoir serving as a mobile phase for chromatography and/or as electrophoresis buffer.

As illustrated in Fig. 4, an electric field may be provided between the first electrode 41 in the first layer 10 and the second electrode 42 in the third layer 30, particularly by connecting the contact tabs 41a, 42a to opposite poles of an external voltage source to electrophoretically move components of the biological sample S from the first layer 10 to the second layer 20 and the third layer 30. In particular, a negative potential may be applied to the first electrode 41 and a positive potential may be applied to the second electrode 42 as depicted in Fig. 4. In particular when the biological sample S is dissolved in a buffer having a pH range between about 6 and about 8, most biomolecules (DNA, RNA and proteins) in the biological sample S are negatively charged. Therefore, most biomolecules will electrophoretically migrate toward the second electrode 42 in the third layer 30 when the first electrode 41 has a negative potential and the second electrode 42 has a positive potential.

In the setup depicted in Fig. 3-6, the first electrode 41 is a grid electrode stretching over the entire first area 2a of the first layer 10, and the second electrode 42 is formed as an elongated rectangular tab having a smaller cross-sectional area perpendicular to the first axis A1 than the first electrode 41.

Thus, as shown in Fig. 4, the biological sample B is focused while passing through the first layer 10 and the second layer 20 to the third layer 30, as indicated by the sample path SP in Fig. 4. In other words, as a result of the convergence of the electric field due to the smaller second electrode 42, the molecules of the biological sample are concentrated to a smaller partial area of the third layer 30.

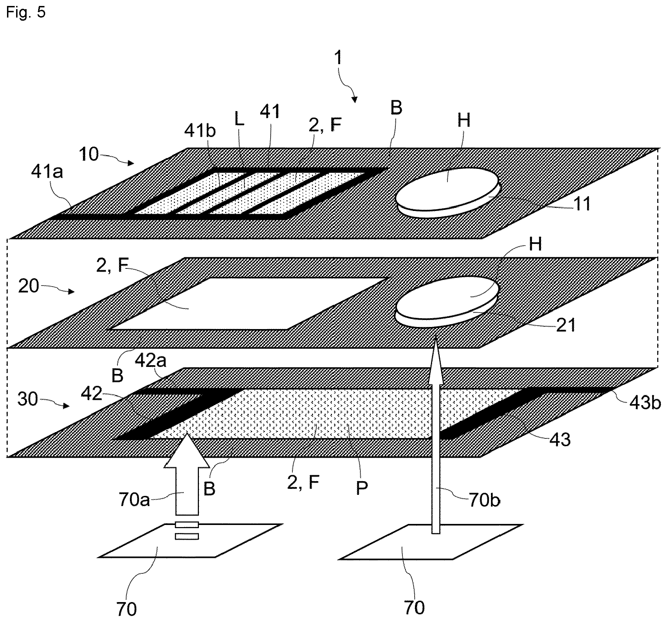

In particular, as illustrated in Fig. 5, the third layer 30 containing the PCR reagents P and the biological sample S is subsequently periodically heated (and optionally cooled) by the heating elements 70 below the third layer 30 to perform a PCR amplification of DNA or RNA contained in the biological sample S (heating of the PCR reagents 70a). To ensure that the third layer 30 does not dry out during the heating cycles of the PCR reaction, the polymer gel H arranged in the opening 11, 21 of the first layer and/or second layer may be activated to release liquid into the third layer 30, in particular by heating with the heating elements 70 (heating of the switchable polymer gel 70b, in case the polymer gel H is temperature-switchable). Of course, the PCR step may be omitted, in particular if analysis of other biomolecules than nucleic acids (e.g. proteins) contained in the biological sample S is desired.

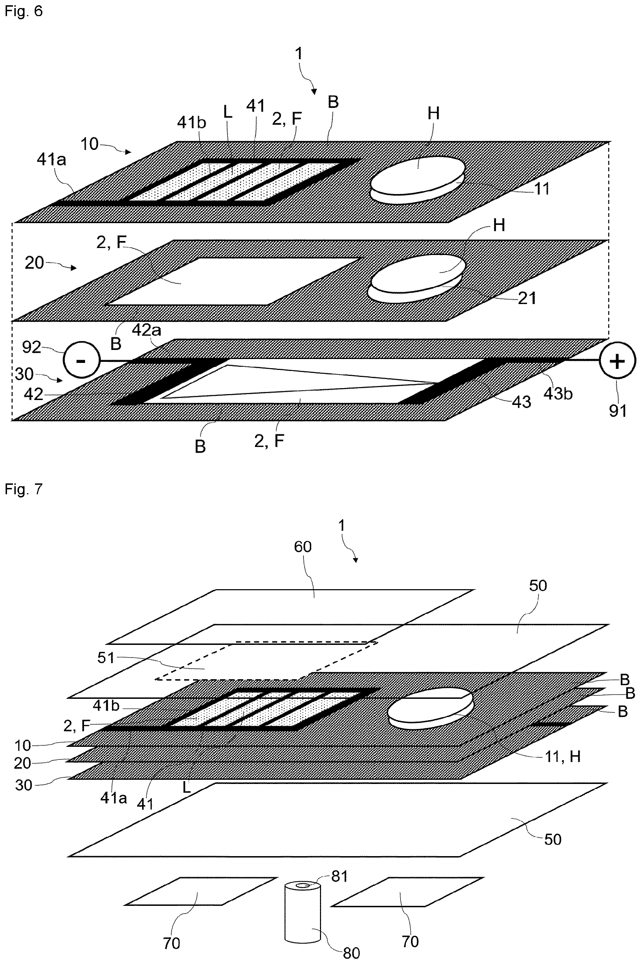

Finally, as shown in Fig. 6, the (particularly previously amplified) molecules contained in the biological sample S may be separated by electrophoresis in the third layer 30. To this end, an electric potential difference is applied between the second electrode 42 and the third electrode 43, such that an electric field is generated in the plane of the third layer 30. In particular, the second electrode 42 may be connected to a negative pole 92 of a voltage source and the third electrode 43 may be connected to a positive pole 91 of the voltage source, such that predominantly negatively charged biomolecules (such as DNA, RNA and proteins) migrate from the second electrode 42 to the third electrode 43.

The voltage source may be configured to provide a constant voltage between the second electrode 42 and the third electrode 43 or a constant current through the third layer 30. During electrophoresis, the current in the third layer 30 may be measured over time using a amperemeter (in case of a constant voltage provided by the voltage source) or the voltage between the second electrode 42 and the third electrode 43 may be measured over time using a voltmeter (in case of a constant current provided by the voltage source, the method of measurement being termed cyclic voltammetry). The resulting time trace of the current or voltage may be used to analyze properties of the biomolecules, particularly nucleic acids, of the biological sample S. As an alternative, the properties of the biomolecules may also be analyzed by coulometry or by an impedance measurement.

Alternatively or additionally, the biomolecules in the biological sample (or amplified from the biological sample) may be analyzed by a marker molecule, which particularly may be disposed in the fibrous material F of the third layer 30 in dry or lyophilized form prior to entry of the biological sample S. In case of nucleic acids, the marker molecule may for instance be a hybridization probe such as a molecular beacon, which is capable of specifically binding to a target DNA or RNA sequence. When proteins are to be detected, the marker molecule may be e.g. an antibody, which specifically binds to an epitope in the protein of interest. The marker molecule may be coupled (e.g. covalently bound) to an optically detectable molecule, such as a dye or a fluorophore, which changes color or emits luminescence or fluorescence light upon specific binding of the marker to the biomolecule of interest. Alternatively, the marker may also comprise one or more substance(s) which undergoe(s) a chemical reaction resulting in a change of color or emission of fluorescent or luminescent light. In case of fluorescence, the device may be illuminated with light of wavelengths overlapping with the absorbance spectrum of the fluorophore, e.g. by an external light source.

To detect the light emitted by the marker, an external optical detector 80 may be used. An example of this is depicted in Fig. 7, where the optical detector 80 is a camera having a lens 81. In this case, the device 1 may be at least partially transparent to maximize the amount of detected light. To this end, for example, the cover layers 50 and/or cover 60 may be formed from a transparent material.

The device 1 and particularly the applied marker M may be configured, such that the emitted light may be detected by an off-the-shelf camera of a mobile device, such as a smart phone or a tablet computer. This improves usability of the device 1, particularly for applications outside of a laboratory, such as on-site analysis of environmental samples.

As an alternative to optical detection, marker molecule may also be detectable by its redox properties.

Of course, the described analysis of biomolecules in the biological sample S may be performed with and without previous electrophoresis.

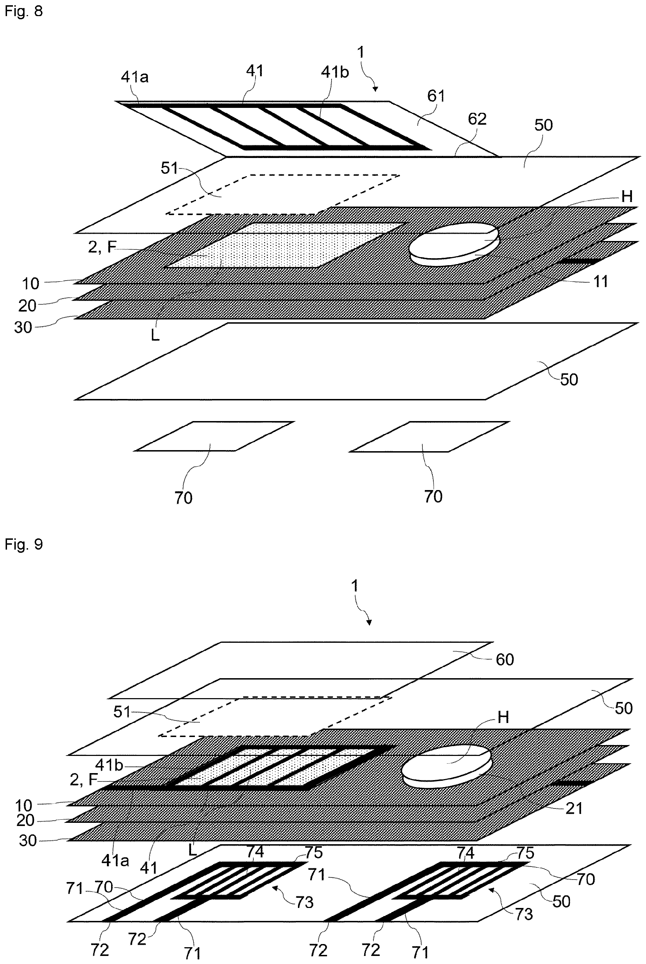

Fig. 8 shows a further embodiment of a device 1 according to the invention. The device 1 differs from the device shown in Fig. 1, in that the device comprises a lid 61 instead of the cover 60, wherein the first electrode 41 (grid electrode) is arranged in or on the lid 61. The lid 61 may be attached to the upper cover layer 50 by a hinge 62. In particular, the first electrode 41 is printed onto the lid 61 (i.e. by applying electrically conductive ink). When the lid 61 is closed, the first electrode 41 is placed directly on the first area 2a of the first layer 10, such that electrophoresis of the biological sample S may be initiated by applying a potential difference between the first electrode 41 and the second electrode 42.

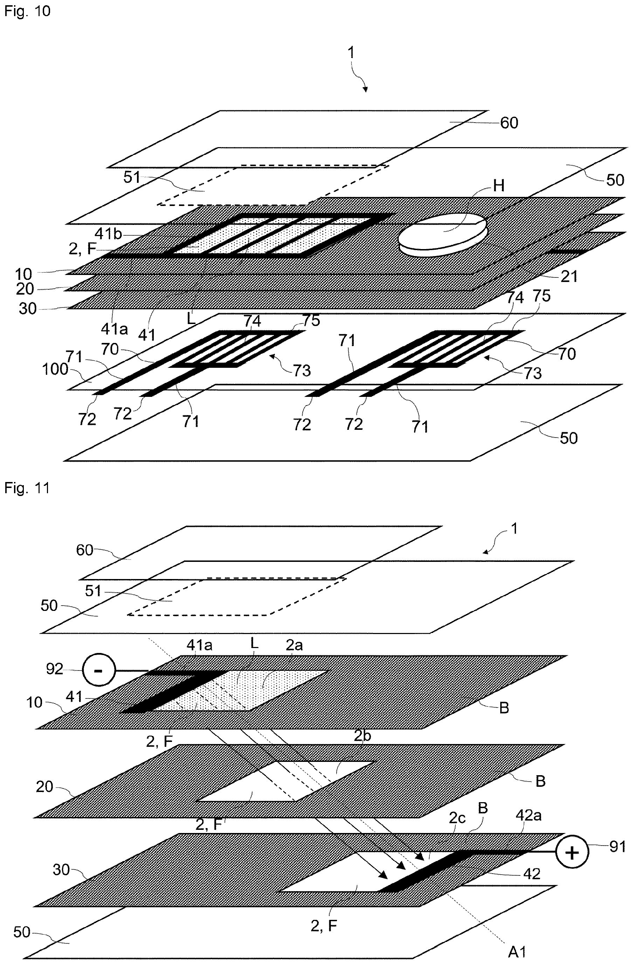

Fig. 9 and 10 show embodiments of the device 1, where the heating elements 70 are formed as resistance heaters, more specifically as heating electrodes. The heating electrodes may be arranged on, particularly printed on (i.e. by applying an electrically conductive ink), the lower cover layer 50 (Fig. 9) or alternatively a further fourth layer 100 placed between the third layer 30 and the lower cover layer 50 (Fig. 10).

The heating electrodes comprise a grid 73 composed of parallel rods 74 surrounded by a rectangular frame 75 and contact tabs 71 connecting the grid 73 to an edge of the respective layer, where they can be connected at ends 72 to the poles of a voltage source to generate a current through the heating electrodes and generate heat due to the electrical resistance of the heating electrodes.

Such integrated heating electrodes obviate the need for an external heating device.

Fig. 11 shows a further device 1 according to the invention having a first layer 10, a second layer 20 and a third layer 30 of a fibrous material F as well as cover layers 50 and a cover 60. The device 1 is particularly identical to the embodiment shown in Fig. 1, except for the following differences: the first layer 10 comprises instead of a grid electrode a first electrode 41 shaped as an elongated tab positioned (in the view of Fig. 11) on the left edge of the first area 2a and the third layer 30 comprises only the second electrode 42 which is positioned on the right edge of the third area 2c. Furthermore, the second area 2b of the second layer 20 is off-set to the right in respect of the first area 2a of the first layer 10, and the third area 2c of the third layer 30 is offset to the right in respect of the second area 2b of the second layer 20. Thereby, the resulting sample path SP or channel formed by the fibrous material F of the first layer 10, second layer 20 and third layer 30 along the first axis A1 extends diagonally in respect of the vertical axis between the first electrode 41 and the second electrode 42. In particular, to move and electrophoretically separate negatively charged molecules in the biological sample, the first electrode 41 may be connected to a negative pole 92 of a voltage source and the second electrode 42 may be connected to a positive pole 91 of a voltage source. This results in a separation of the biological sample, whereby bands of molecules in the biological sample are aligned next to each other in a projection along the plane parallel to the second area 2b when the bands are detected optically.

**List of reference numerals**

| | |
|---|---|
| 1 | Device for analyzing biological samples |
| 2 | Substrate |
| 2a | First area |
| 2b | Second area |
| 2c | Third area |
| 10 | First layer |
| 11 | Opening |
| 20 | Second layer |
| 21 | Opening |
| 30 | Third layer |
| 41 | First electrode |
| 41a | Contact tab |
| 41b | Grid stucture |
| 41c | Rod |
| 41d | Frame |
| 42 | Second electrode |
| 42a | Contact tab |
| 43 | Third electrode |
| 43a | Contact tab |
| 50 | Cover layer |
| 60 | Cover |
| 61 | Lid |
| 62 | Hinge |
| 70 | Heating element |
| 70a | Heating of the PCR reagents |
| 70b | Heating of the polymer gel |
| 71 | Contact tab |
| 72 | End |
| 73 | Grid |
| 74 | Rod |
| 75 | Frame |
| 80 | Optical detector |
| 81 | Lens |
| 91 | Positive pole |
| 92 | Negative pole |
| 100 | Fourth layer |
| A1 | First axis |
| A2 | Second axis |
| B | Barrier structure |
| F | Fibrous material |
| H | Polymer gel |
| L | Lysis agent |
| P | PCR reagents |
| S | Biological sample |
| SP | Sample path |

## Claims

1. A device (1) for analyzing biological samples (S) comprising
- a substrate (2) for receiving a biological sample (S), wherein the substrate (2) comprises or consists of a fibrous material (F) configured to form a stationary phase which retains molecules in a biological sample (S) dissolved in a liquid mobile phase depending on molecular weight and/or polarity of the molecules,
- a first electrode (41) and a second electrode (42), which are arranged along a first axis (A1) and configured to generate an electric field acting along the first axis (A1) when an electric potential difference is provided between the first electrode (41) and the second electrode (42), so that charged molecules contained in the biological sample (S) are movable through the substrate (2) along the first axis (A1) and/or separable by their molecular weight, their polarity and/or their charge,
**characterized in that**
the substrate (2) comprises a chemical lysing agent (L) capable of lysing the biological sample (S).

2. The device (1) according to claim 1, **characterized in that** the lysing agent (L) is disposed within the substrate (2) in dry form, wherein the lysing agent (L) is dissolvable upon applying a liquid biological sample (S) to the substrate (2), such that the biological sample (S) is lysed.

3. The device (1) according to claim 1 or 2, **characterized in that** the substrate (2) comprises a barrier structure (B) which is impermeable to the liquid mobile phase, wherein the barrier structure (B) forms at least one channel which is permeable to the liquid mobile phase in the substrate (2) limited by the barrier structure (B).

4. The device (1) according to any one of the preceding claims, **characterized in that** the device (1) comprises a third electrode (43), wherein the second electrode (42) and the third electrode (43) are arranged along a second axis (A2) which is non-parallel to the first axis (A1) and configured to generate an electric field acting along the second axis (A2) when an electric potential difference is applied between the second electrode (42) and the third electrode (43).

5. The device (1) according to any one of the preceding claims, **characterized in that** the first electrode (41), the second electrode (42) and/or the third electrode (43) is embedded into the fibrous material (F) of the substrate (2) or connected to the fibrous material (F) of the substrate (2), particularly wherein the first electrode (41), the second electrode (42) and/or the third electrode (43) is printed onto the substrate (2).

6. The device (1) according to any one of the preceding claims, **characterized in that** the second electrode (42) comprises a cross-sectional area perpendicular to the first axis (A1), which is smaller than a cross-sectional area perpendicular to the first axis (A1) of the first electrode (41), so that charged molecules in the biological sample (S) are focused in the substrate (2) while moving through the electric field between the first electrode (41) and the second electrode (42).

7. The device (1) according to any one of the preceding claims, **characterized in that** the first electrode (41) forms a grid structure (41b).

8. The device (1) according to any one of the preceding claims, **characterized in that** a DNA polymerase and PCR primers are disposed in the substrate (2), so that a DNA sequence of the biological sample (S) can be amplified by PCR by means of the DNA polymerase if the PCR primers contain complementary DNA sequences to said DNA sequence.

9. The device (1) according to any one of the preceding claims, **characterized in that** the device (1) comprises a polymer gel (H), particularly a hydrogel, in contact with the substrate (2), so that liquid is exchangeable between the polymer gel (H) and the substrate (2), particularly wherein the polymer gel (H) is switchable by setting a physical parameter, particularly changing a temperature, a pH, or an ionic strength, providing electromagnetic radiation, or initiating a chemical reaction, so that liquid is exchanged between the polymer gel (H) and the substrate (2).

10. The device (1) according to any one of the preceding claims, **characterized in that** the device (1) comprises a heating element (70) for heating, particularly periodic heating, of the substrate (2), particularly wherein the heating element (70) is formed as a heating electrode, wherein the heating electrode is connected to an outside of the device (1) via electrically conductive contact tabs (71), wherein ends (72) of the first and second contact tabs (71) are configured to be electrically connected to the poles of a voltage source, so that an electric current flows through the heating electrode thereby heating the substrate (2) by its electric resistance.

11. The device (1) according to any one of the preceding claims, **characterized in that** the substrate (2) comprises a marker for labeling molecules contained in the biological sample, particularly wherein the marker comprises a dye or wherein the marker is detectable by its redox properties.

12. The device (1) according to any one of the preceding claims, **characterized in that** the device is at least partially transparent, so that the molecules contained in the biological sample (S) can be analyzed by an optical detector (80), particularly a camera, more particularly a camera contained in a handheld device such as a smart phone or a tablet computer.

13. The device (1) according to any one of the preceding claims, **characterized in that** the device (1) comprises a plurality of layers (10, 20, 30) comprising said fibrous material (F), wherein the layers (10, 20, 30) are arranged on top of each other, so that the fibrous material (F) of the layers (10, 20, 30) forms the substrate (2), wherein the layers (10, 20, 30) extend in respective planes which are non-parallel to the first axis (A1), so that charged molecules contained in the biological sample (S) are movable through the layers (10, 20, 30) by means of the electric field acting along the first axis (A1) between the first electrode (41) and the second electrode (42), particularly wherein the planes in which the layers (10, 20, 30) extend, are parallel to the second axis (A2), so that the charged molecules contained in the biological sample (S) are movable in the respective layer (10, 20, 30) and separable by their molecular weight, polarity and/or charge.

14. The device (1) according to claim 13, **characterized in that** the device (1) comprises a first layer (10), wherein the fibrous material (F) of the first layer (10) comprises the lying agent (L), a second layer (20) in contact with the first layer (10), wherein the second layer (20) is configured to filtrate the biological sample (S) when the biological sample (S) passes through the second layer (20), and a third layer (30) in contact with the second layer (20), wherein the third layer (30) comprises said second electrode (42) and said third electrode (43), such that molecules in said biological sample (S) are separable by molecular weight, polarity and/or charge in the third layer (30) using the electric field acting between the second electrode (42) and the third electrode (43), wherein particularly the fibrous material (F) of the third layer (30) comprises said marker and/or said DNA polymerase and said PCR primers, particularly wherein the fibrous material (F) of the third layer (30) is in contact with said heating element (70), particularly wherein said polymer gel (H) is arranged in said first layer (10) and/or said second layer (20).

15. A method for analyzing biological samples (S) by means of the device (1) according to any one of the claims 1-14, wherein
a. a biological sample (S) is applied to the substrate (2), such that the biological sample (S) is in contact with said lysing agent (L), resulting in lysis of the biological sample (S);
b. an electric potential difference is provided between the first electrode (41) and the second electrode (42), such that the biological sample (S) moves through the substrate (2) and is separated by its molecular weight, polarity and/or charge;
c. optionally, the substrate (2) is periodically heated, such that a DNA sequence of the biological sample (S) is amplified by PCR in the substrate (2);
d. optionally, an electric potential difference is provided between the second electrode (42) and the third electrode (43), such that the biological sample (S) is separated by its molecular weight, polarity and/or charge, particularly wherein the current or the voltage between the second electrode (42) and the third electrode (43) is measured over time, wherein properties of the biological sample (S) are derived from the measured current or voltage; and
e. optionally, the biological sample (S) is analyzed by detecting the marker in the substrate (2).
